# EUROPEAN PATENT APPLICATION

(11) **EP 0 708 119 A1**
(43) Date of publication of application: **24.04.1996**
(21) Application number: 95116352.6
(22) Date of filing: 17.10.1995
(51) Int. Cl.: C08F 220/04, A61L 15/60

(54) **Self-crosslinking, aqueous absorbent polymer compositions**

(30) Priority: 21.10.1994 US 326890; 05.04.1995 US 417320; 07.04.1995 US 418304
(71) Applicant: AIR PRODUCTS AND CHEMICALS, INC., Allentown, PA 18195-1501 (US)
(72) Inventor: Tze-Chiang Chemg, John, Allentown, PA 18103 (US); Koltisko, Bernard M., Jr., Emmaus, PA 18049 (US); Marten, Finn L., Emmaus, PA 18049 (US); Goldstein, Joel E., Allentown, PA 18104-2903 (US)
(74) Representative: Schwabe - Sandmair - Marx

(57) **Abstract**

An aqueous polymer composition comprising 10 to 50 wt% of a polymer in an aqueous medium, the polymer demonstrating a maximum absorbency capacity of at least 10 g of a 1% aqueous saline solution/g polymer and consisting essentially of 25-90 wt% (4, 5),''1% α,β-ethylenically unsaturated carboxylic acid monomer, 0-50 wt% (meth)allylsulfonic acid, crosslinking comonomer comprising 0.1-10 wt% latent crosslinking comonomer or 0.01-2 wt% active crosslinking comonomer, or both, and at least one softening monomer in a sufficient amount so that the polymer has a Tg of <140°C, the softening monomer characterized as having a homopolymer that exhibits a Tg≦35°C, the aqueous composition having a viscosity of less than about 3000 mPa and being adjusted to pH 4-7 with alkali or alkaline earth metal hydroxide. Such aqueous compositions can be applied to nonwoven and woven substrates or loose cellulosic fibers to make superabsorbent products.

## Description

### CROSS-REFERENCE TO RELATED APPLICATIONS

This application is a continuation-in-part of application Serial No. 08/417,320 filed on 5 April 1995 which is a continuation-in-part of application Serial No. 08/326,890 filed on 21 October 1994, and is also a continuation-in-part of application Serial No. 08/418,304 filed on 7 April 1995 which is a continuation-in-part of application Serial No. 08/326,890 filed on 21 October 1994, all of which are hereby incorporated by reference.

### TECHNICAL FIELD OF THE INVENTION

The invention relates to certain polymers characterized by their ability to absorb a large amount of water, which polymers are generally referred to as superabsorbing polymers.

### BACKGROUND OF THE INVENTION

Superabsorbing polymers find application in the medical industry, the food industry and agriculture and, further, are used in many consumer products. Superabsorbing polymers are of great interest in personal hygiene products such as diapers, incontinent pads, and feminine care products. Normally, they are used in their usual commercial granular or powdered form, in mixtures with various cellulosic fibers. Those fibers, for example, wood pulp or modified cellulose fibers, when used alone, have certain shortcomings in that the fluid is held mainly in the interstices between individual fibers and can be readily squeezed out under pressure, and the mechanical strength of the fibers is significantly reduced when wet. Because of that, cellulosic fibers by themselves are not ideal absorbent materials for such applications. The addition of superabsorbing polymers to the pulp improves the retention of fluid under pressure and gives a blend with increased absorptive capacity.

Superabsorbing polymers swell to a large degree in contact with water, but do not fully dissolve. They are usually prepared by either one of two methods, namely, crosslinking water-soluble polymers to make them water insoluble, while allowing them to retain their ability to swell in water; or modifying water-insoluble polymers by introducing hydrophilic groups to cause swelling in contact with water. The first method is the one most commonly used to prepare superabsorbing polymers. The crosslink density is of critical importance since too little crosslinking produces a soft, loose gel, which still is quite water soluble, while too much crosslinking reduces polymer swelling and thus its ability to absorb water.

The commercial absorbent materials are primarily based on the powdered form of sodium acrylate/acrylic acid polymers or copolymers. One of the shortcomings for the powder material is that the hard granular particles require nonwoven manufacturers to subject their web to another operation to add the powder to the composite for personal care applications. As another shortcoming, the prior art commercial superabsorbing polymers normally do not have sufficient tack to adhere to the cellulosic fibers with which they are blended. As a result, they tend to separate from the cellulosic fibers and migrate within the pad or sift out of the pad during handling; i.e., these are problems of uneven distribution and shifting of particles due to lack of adhesion to the substrate. Obviously, it would be desirable to fix the superabsorbing polymer in place in the pad so that the polymer does not migrate.

WO 90/08806 discloses superabsorbing copolymers of vinyl alcohol which are made by alcoholysis, in the presence of a small amount of an alkali metal alkoxide or hydroxide, of a precursor copolymer VAc/X/Y, where VAc stands for vinyl acetate; X is a dialkyl ester of one of specified ethylenically unsaturated dicarboxylic acids; and Y is a copolymerizable ethylenically unsaturated monocarboxylic acid.

U.S. 4,320,040 discloses a method for producing a hydrophilic gel comprising polymerizing acrylic acid and/or methacrylic acid, or a neutralized product thereof, in the presence of polyvinyl alcohol to obtain a polymer which is subjected to heat treatment, wherein the polymerization is carried out in an aqueous solution having a total concentration of acrylic acid and/or methacrylic acid or its neutralized product and polyvinyl alcohol of 10-60% by weight, the heat treatment temperature being 50 to 150°C and the weight ratio of polyvinyl alcohol to acrylic acid and/or methacrylic acid or its salt being 5-95:95-5.

U.S. 4,743,244 discloses a polymer composition which is water-absorbent upon curing comprising:
(a) a copolymer containing from about 25 to 75 mole% of at least one α,β-unsaturated monomer bearing at least one carboxylic acid unit; and
(b) a polyether derived from C₂ to C₁₀ alkylene oxides.

U.S. 4,683,274 discloses a water-absorbent resin produced by subjecting an aqueous solution of an α,β-unsaturated carboxylic acid and an alkali metal salt thereof to polymerization with a radical polymerization initiator in a petroleum-based hydrocarbon solvent in the presence or absence of a crosslinking agent and, in this polymerization, using a saccharose-fatty acid ester as a protective colloid agent.

U.S. 4,735,987 discloses a method for the manufacture of a high-expansion type absorbent polymer characterized by causing an absorbent polymer which results from the polymerization of a monomer and an alkali salt of acrylic acid to be crosslinked, during azeotropic dehydration thereof, with a crosslinking agent possessing two or more functional groups in the presence of an inorganic substance and the drying of the crosslinked absorbent polymer.

U.S. 4,666,983 discloses an absorbent article obtained by mixing 100 parts by weight of an absorbent resin powder, having a carboxyl group with 0.001 to 10 parts by weight of a crosslinking agent having at least two functional groups capable of reacting with the carboxyl group per molecule and reacting the absorbent resin powder with the crosslinking agent to crosslink the molecular chains existing at least in the vicinity of the surface of the absorbent resin powder.

U.S. 5,192,617 discloses cross-linked polymeric compositions capable of forming continuous matrices for liquid absorbent, semi-interpenetrating polymer networks.

U.S. 4,975,320 discloses nonwoven products bonded with binder emulsions of copolymers or vinyl acetate/ethylene/incompatible comonomer/latent crosslinking comonomer.

U.S 3,926,891 discloses water swellable polyacrylates made by saponifying a polyacrylate comprising alkyl acrylate, carboxylic acid comonomer and optionally hydroxyalkyl methacrylate.

U.S. 4,057,521 discloses a water swellable polymer containing 25 to 98 wt% alkali metal salt of a monosulfonic or monocarboxylic acid monomer, 2 to 50 wt% monocarboxylic acid monomer and 0.1 to 5.0 wt% N-substituted (meth)acrylamide.

U.S. 4,062,817 discloses water absorbent polymers comprising carboxylic acid monomer, (meth)acrylic esters containing alkyl groups of 10 to 30 carbons, (meth)acrylic esters containing alkyl groups of 1 to 8 carbons, and optionally a small amount of a crosslinking agent.

U.S. 4,190,562 discloses water absorbent polymers comprising carboxylic acid monomer, (meth)acrylic esters containing alkyl groups of 10 to 30 carbons, (meth)acrylic nitrile or amide, and optionally a small amount of a crosslinking agent.

U.S. 4,654,039 discloses hydrogel-forming polymer compositions comprising substantially water-insoluble, slightly crosslinked, partially neutralized polymers which are prepared from unsaturated polymerizable, acid group-containing monomers and crosslinking agents.

U.S. 4,741,790 discloses aqueous adhesives containing water swellable polymers comprising ethoxylated comonomers capped with a hydrophobic group.

U.S. 4,861,539 discloses water absorbent, water insoluble, crosslinked polymer fiber or film made by the dry extrusion of a solution of a substantially linear polymer formed from a water soluble blend of monomers comprising a plasticizing monomer.

U.S. 4,892,916 discloses polymers comprising an ionic monomer, a substantially nonionic monomer, optionally a crosslinking agent, and an allyl ether containing a hydrophobic group.

U.S. 4,962,172 and 5,280,079 disclose water absorbent, water insoluble, substantially linear polymers prepared from water soluble monomer blends comprising carboxylic and hydroxylic monomers.

U.S. 4,980,434 discloses water absorptive polymers comprising water soluble monomers, crosslinking agent and an ethoxylated monomer containing a hydrophobic group.

U.S. 5,147,956 discloses water soluble, substantially linear polymers comprising carboxylic acid monomer and an alkoxylated allyl or acrylate monomer.

### SUMMARY OF THE INVENTION

The present invention provides an aqueous absorbent polymer composition comprising a carboxylate-containing polymer in an aqueous medium which has been adjusted with an alkali or alkaline earth metal hydroxide to pH 4 to 7. The polymer demonstrates a maximum absorbency capacity of at least 10 grams of a 1% aqueous saline solution per gram of polymer and consists essentially of 25-90 wt% α,β-ethylenically unsaturated carboxylic acid monomer, 0-50 wt% allyl or methallyl sulfonic acid monomer, or both, crosslinking comonomer comprising 0.1-10 wt% latent crosslinking comonomer or 0.01-2 wt% active crosslinking comonomer, or both, and at least one softening monomer in an amount effective to yield a polymer having a Tg <140° C. The aqueous absorbent polymer composition has a viscosity less than about 3000 mPa (20% solids), preferably less than about 2000 mPa, and most preferably in the range of 500 to 1500 mPa. The viscosity of the aqueous composition is measured with a Brookfield viscometer using spindle #4 at 60 rpm and 23°C.

As an advantage of the invention, the carboxylate-containing polymer may be applied to the absorbent core product, namely loose cellulosic fibers or a nonwoven or woven web, in liquid form, i.e., as an aqueous solution or dispersion, in a single step operation, thus resulting in a more uniform and consistent layer of polymer on the fibers or web. In addition, the polymer remains in place on the substrate.

As another embodiment of the invention there are also provided superabsorbent fibers comprising fibers containing a sufficient amount of adhered superabsorbing polymer to provide fibers having enhanced absorbent properties.

The polymer not only possesses sufficient tack to adhere to the fibers of the nonwoven web but also has sufficient adhesive strength to act as a binder for the nonwoven web. The application of heat causes crosslinking of the polymer containing latent crosslinking comonomer to afford the superabsorbent property.

Therefore, as an embodiment of the invention there is also provided a nonwoven web of fibers bonded together with a sufficient amount of the superabsorbing polymer composition to provide a self-sustaining superabsorbent web.

As another embodiment of the invention there is also provided a nonwoven web of fibers bonded together with a sufficient amount of a binder polymer to provide a self-sustaining web and also a sufficient amount of the superabsorbing polymer composition to provide a superabsorbent web.

The incorporation of (meth)allyl sulfonic acid monomer into the polymerization recipe provides for degradative chain transfer which results in low molecular weight polymers, low viscosity aqueous polymer media and sulfonate functionality in the polymer backbone for improving the rate of absorbency.

### DETAILED DESCRIPTION OF THE INVENTION

The liquid absorbent polymer composition of the invention as a solution or emulsion comprises an aqueous medium, e.g., water, of 10 to 50 wt% solids, preferably 20 to 45 wt%, of a polymer prepared by aqueous solution or emulsion polymerization of the monomers in the presence of a stabilizing system comprising at least one polyvinyl alcohol (PVOH) or at least one surfactant, or mixture thereof. The polymer consists essentially of an α,β-ethylenically unsaturated carboxylic acid monomer, a latent crosslinking comonomer and/or active crosslinking comonomer, and one or more softening monomers such as, for example, ethylene, vinyl alkanoates, mono- and diesters of dicarboxylic acid monomers such as mono- and dialkyl maleates or fumarates, and alkyl acrylates and methacrylates. When the polymer also contains an allyl and/or a methallyl sulfonic acid monomer, the liquid absorbent polymer composition preferably comprises 35 to 45 wt% solids. When PVOH is used in the stabilizing system, the monomers become grafted onto or complexed with the PVOH backbone.

Suitable carboxylic acid and acid anhydride monomers which compose 25 to 90 wt%, preferably 35 to 70 wt%, of the polymer may be any one or more of the α,β-ethylenically unsaturated mono- or dicarboxylic acids and acid anhydrides, such as acrylic acid, methacrylic acid, crotonic acid, maleic acid/anhydride, itaconic acid, fumaric acid and the like. The preferred carboxylic acid monomer for use in the present invention is acrylic acid.

The other acid-containing comonomers, optionally, but preferably used in the copolymer at 2 to 50 wt%, most preferably 5 to 20 wt%, comprise allyl sulfonic acid and/or methallyl sulfonic acid. These acid comonomers, which function as chain transfer agents, are preferably polymerized into the polymer using the corresponding sodium salt which results in solutions or emulsions with relatively high solids content and low viscosity. Contemplated as the functional equivalent of the (meth)allyl sulfonic acids for the purpose of the present invention are the (meth)allyl sulfuric acids, the (meth)allyl phosphoric acids and the (meth)allyl alcohols.

As the crosslinking comonomer, the polymer may contain 0.1-10 wt% latent crosslinking comonomer, preferably 1-5 wt%. For purposes of this invention, the term "latent crosslinking comonomer" means a functional vinyl comonomer in which the functionality causes crosslinking of the polymer upon the subsequent application of energy, generally by applying heat, for example, by drying and curing of the polymer, often in the presence of a catalyst, or by applying radiation The latent crosslinking monomer provides a thermosetting characteristic to the polymer. Upon the subsequent application of energy, the latent crosslinking comonomer forms an insoluble crosslinked network. Examples of suitable latent crosslinking comonomers are N-methylol acrylamide (NMA), acrylamidoglycolic acid (AGA), methylacrylamidoglycolate methyl ester (MAGME), acrylamidobutyraldehyde dialkyl acetal (ABDA), such as the dimethyl or diethyl acetals, isobutoxy methyl acrylamide (IBMA), chlorohydroxypropylmethacrylate, acetoacetoxyethyl (meth)acrylate (AAEA or AAEM) and 1-(1-isocyanato-1-methylethyl)-4-(1-methylethenyl) benzene. NMA and AAEM are the preferred crosslinking comonomers.

If the polymer contains an active crosslinking comonomer, it is present at 0.01-2 wt%, preferably 0.05-1 wt%. The term "active crosslinking comonomer" means a polyethylenically unsaturated comonomer which immediately provides crosslinking and branching of the polymer during the polymerization step to increase the molecular weight of the polymer. Subsequent drying and heating or other curing techniques are not required for the crosslinking and branching of the polymer by the active crosslinking comonomer. The following compounds are illustrative of suitable active crosslinking comonomers: methylenebisacrylamide, alkylene glycol di(meth)acrylates such as ethylene glycol diacrylate and triethylene glycol dimethacrylate, 1.1.1-trimethylolpropane dimethacrylate, divinyl benzene, vinyl (meth)acrylate, divinyl adipate, divinyl ether, triallyl (iso)cyanurate, allyl (meth)acrylate, diallyl maleate, diallyl fumarate and others known in the polymerization art. The preferred active crosslinking comonomers are methylenebisacrylamide and ethylene glycol di(meth)acrylate.

The polymer also contains one or more softening monomers to provide the polymer and resulting absorbent product with drape and flexibility. Such monomers are used in amounts sufficient to yield a polymer having a Tg <140°C, preferably a Tg ranging from -20 to +120°C. The Tg is determined on a polymer which has been neutralized with aqueous sodium hydroxide to a pH of about 5-5.5, i.e., about 75% neutralized.

"Softening monomers" are those copolymerizable monomers whose respective homopolymers exhibit a Tg≦35°C. Such softening monomers would include ethylene, vinyl alkanoates, alkyl acrylates and methacrylates and mono- and diesters of dicarboxylic acid monomers.

Suitable vinyl alkanoates include any of the C₁-C₁₂ esters of vinyl alcohol, such as vinyl formate, vinyl propionate, vinyl butyrate, vinyl versatate, and preferably vinyl acetate. These monomers may compose 5-75 wt%, preferably 10-30 wt%, of the polymer. Vinyl acetate homopolymer has a Tg of ∼32°C.

As to softening monomers which are mono- or diesters of dicarboxylic acid monomers and may compose 2-50 wt%, preferably 4-20 wt%, of the polymer, they may be a mono- or dialkyl maleate or fumarate such as any mono- or diester of maleic acid or fumaric acid with a C₁-C₁₆ alkanol (monoalcohol), preferably a C₄-C₈ alkanol, i.e., octyl alcohol, isooctyl alcohol, butyl alcohol, isobutyl alcohol, amyl alcohol, tridecyl alcohol and the like. The preferred comonomer is dioctyl maleate.

Alkyl (meth)acrylates which may be softening monomers are those esters of acrylic or methacrylic acid with a C₂-C₈ alkanol (monoalcohol) such as butyl acrylate, butyl methacrylate, hexyl acrylate, preferably 2-ethylhexyl acrylate, and the like. The homopolymer of 2-ethylhexyl acrylate, for example, has a Tg of -70°C. Alkyl (meth)acrylates may compose 2-50 wt%, preferably 4-20 wt%, of the polymer.

The monomers can be polymerized in an aqueous medium in the presence of at least one PVOH which is 80-99 mole% hydrolyzed and has a degree of polymerization (DPn) ranging from 150 to 1500, preferably 200 to 800. It is preferred to use a PVOH which is partially (87-89 mole%) hydrolyzed with a DPn of 200 to 800. The PVOH, when used, is used at 1-10 wt%, preferably 2-8 wt%, based on copolymerizable monomers. Of course, it is possible to use a combination of PVOH's. It is believed that the PVOH acts as the site, or backbone, for the graft polymerization or complexation of the comonomers.

In addition to, or in place of, the PVOH composing the stabilizing system for the aqueous polymerization reaction, emulsifying agents, or surfactants, can be used. Emulsifying agents composing the stabilizing system which can be used in the polymerization recipe include those ionic and nonionic surfactants well known in the aqueous polymerization art, preferably the anionic types. The concentration range of the total amount of the emulsifying agents useful is from 0.1 to 10%, based on total emulsion.

The aqueous composition of the carboxylic acid-containing polymer contains 10 to 50 wt%, preferably 20 to 45 wt%, polymer solids and, when the polymer advantageously contains allyl sulfonic acid and/or methallyl sulfonic acid, the aqueous composition desirably contains about 30 to 50 wt%, preferably 35 to 45 wt%, polymer solids. It is adjusted to a pH of about 4-7 using an alkali metal hydroxide, such as, for example, sodium hydroxide or potassium hydroxide, or an alkaline earth metal hydroxide, such as, for example, calcium hydroxide. It is preferred to use sodium or potassium hydroxide. (Obviously, a metal alkoxide can be use in place of the metal hydroxide.) It is also preferred to adjust the aqueous composition to about pH 5 to 6.

It is desirable that polymers for making the superabsorbent product yield an aqueous solution or dispersion having a sufficiently low viscosity so it can be applied to the substrate, e.g., loose fibers or nonwoven web, as a spray. Suitable viscosities would be <3000 mPa, preferably <2000 mPa, and most preferably 500 to 1500 mPa. The Brookfield viscosity is determined on the aqueous polymer composition at 20% solids and 23°C using a spindle #4 at 60 rpm. If the polymer contains allyl sulfonic acid and/or methallyl sulfonic acid, it is preferred that the aqueous solution or dispersion has a Brookfield viscosity <3000 mPa, preferably <2000 mPa, and most preferably 500 to 1500 mPa, determined on the aqueous polymer composition at 40% solids and 23°C using a spindle #4 at 60 rpm.

Any free radical generating source, such as peroxides and persulfates, may be used to initiate the polymerization of the monomers and carry out the polymerization reaction. Combination-type systems employing both reducing agents and oxidizing agents well known in the art, i.e., redox systems, can also be used. However, it is preferred to use an alkali metal persulfate such as potassium persulfate.

When reference is made to incremental addition, whether of vinyl acetate, any comonomer or free radical source, substantially uniform additions, both with respect to quantity and time, and intermittent additions are contemplated. Such additions are often referred to as "delay" additions.

Suitable aqueous polymer compositions can be prepared by polymerizing the monomers in the presence of an aqueous solution of PVOH. Obviously when ethylene is used as a softening comonomer, the polymerization is performed under ethylene pressure using pressure reactors as is well known in the art. Substantially all of the PVOH in water and a portion of the softening monomer(s), e.g., vinyl acetate, and acrylic acid are initially charged to the polymerization vessel. Most advantageously, at least about 10 to 30 wt% and preferably at least about 15 wt% of the total vinyl acetate to be polymerized is initially charged to the reactor. Similarly, at least about 10 to 50 wt% and preferably at least about 20 wt% of the total acrylic acid to be polymerized is initially charged to the reactor. Likewise, at least about 30 to 80 wt% and preferably at least about 50 wt% of the total (meth)allylsulfonic acid to be polymerized is initially charged to the reactor. It is also contemplated that all of the (meth)allylsulfonic acid to be polymerized is initially charged to the reactor. The aqueous composition is then adjusted to pH 4-5 with alkali metal hydroxide solution. The remaining amounts of the vinyl acetate, acrylic acid and (meth)allylsulfonic acid are desirably delay additions. The entire amount of the softening monomers may desirably be added initially (up front). If present, the active crosslinking comonomer and the latent crosslinking monomer may be added all at once or the latent crosslinking comonomer may be added incrementally over the course of the polymerization reaction.

The polymerization reaction is performed at temperature ranging from 55° to 75°C, preferably about 70°C, when using persulfate initiators.

More specifically, an aqueous solution containing the PVOH is initially prepared. At least 50 wt% of the (meth)allylsulfonic acid monomer and at least 10 wt% of the carboxylate-containing monomer, are then added and the solution is adjusted with an alkali metal hydroxide or an alkaline earth metal hydroxide to a pH of about 4.5. At least 10 wt% of the vinyl acetate and all of the dialkyl maleate or fumarate is then added to the aqueous medium. Polymerization is initiated by the addition of the free radical source. After the initiation a delay feed of the remaining carboxylic acid containing monomer, the remaining other monomers, crosslinking agent and additional free radical source are added as delays. The polymerization reaction is generally continued until the residual vinyl acetate and acrylic acid monomer content is below about 0.5%.

The resulting aqueous polymer composition is treated with an aqueous solution of alkali or alkaline earth metal hydroxide or alkoxide until the pH is about 4-7, preferably 5-5.5. The polymer demonstrates a maximum absorbency capacity of at least 10 grams of a 1% aqueous saline solution per gram of polymer, preferably a maximum absorbency capacity of at least 15 g aq saline soln/g polymer. The maximum absorbency capacity of the polymer is determined according to the following procedure:

The aqueous composition after pH adjustment to about 5 with 10% NaOH is drizzled onto a preweighed 7 cm disc of Whatman #4 filter paper (about 20% polymer on paper) and dried at 300°F (149°C) for 20 minutes. The sample is placed in a sealed plastic bag in a controlled temperature and humidity room (23°C; 50% relative humidity) overnight. The equilibrated sample is sandwiched between two blank pieces of Whatman #4 filter paper and placed on a Gravimetric Absorbency Test System (GATS) apparatus (from MK Systems) with 0.07 psi weight on it to prevent the sample from floating away.

The aqueous absorbent polymer composition can be applied, for example by spraying, onto loose fibers, preferably cellulosic fibers, to produce a superabsorbent product. The amount of the absorbent polymer applied and adhered to the fibers is that amount sufficient to provide enhanced absorbent properties to the fibers and may range from 51 to 50 wt%, preferably 10 to 25 wt%, of the fibers. The produced superabsorbent fibers may be used in many applications including as the fluff in diapers. Examples of the fibers to be used in making the superabsorbent product are the natural cellulose fibers such as wood pulp, cotton and hemp and the synthetic cellulose fibers such as rayon and regenerated cellulose.

The aqueous absorbent polymer composition can be sprayed, foam coated, printed or dip saturated onto a nonwoven or woven web to afford a superabsorbent product. The nonwoven web can be bonded with polymer binders well known in the art, such as vinyl acetate/ethylene/N-methylolacrylamide (VAE-NMA) copolymers, self-crosslinking acrylics and styrene-butadienes, and the liquid absorbent composition applied thereto, or the polymer composition itself may have sufficient adhesive qualities (tack and wet and dry strength) to use it as both the nonwoven binder and the absorbent material.

Thus, various copolymer binders known in the art can be used to prepare nonwoven products, or fabrics, by a variety of methods known in the art which, in general, involve the impregnation of a loosely assembled mass of fibers with the binder emulsion, followed by a moderate heating to dry the mass. This moderate heating also serves to cure the binder by forming a crosslinked interpolymer. Before the binder is applied, it is, of course, mixed with a suitable catalyst for the crosslinking monomer. For example, an acid catalyst such as mineral acids, e.g., hydrogen chloride, or organic acids, e.g., oxalic acid, or acid salts such ammonium chloride, are suitably used as known in the art. The amount of catalyst is generally from 0.5 to 2% of the total polymer.

The starting fiber layer or mass can be formed by any one of the conventional techniques for depositing or arranging fibers in a web or layer. These techniques include carding, garnetting, air-laying, wet laying and the like. Individual webs or thin layers formed by one or more of these techniques can also be laminated to provide a thicker layer for conversion into a fabric. Typically, the fibers extend in a plurality of diverse directions in general alignment with the major plane of the fabric, overlapping, intersecting and supporting one another to form an open, porous structure.

Examples of the fibers to be used in the starting layer are the natural cellulose fibers such as wood pulp, cotton and hemp and the synthetic cellulose fibers such as rayon, and regenerated cellulose. Often the fiber starting layer contains at least 50% cellulose fibers, whether they be natural or synthetic, or a combination thereof. Often the fibers in the starting layer may comprise natural fiber such as wool, jute; artificial fibers such as cellulose acetate; synthetic fibers such as polyamides, nylon, polyesters, acrylics, polyolefins, i.e., polyethylene, polyvinyl chloride, polyurethane, and the like, alone or in combination with one another.

The fibrous starting layer is subjected to at least one of several types of bonding operations to anchor the individual fibers together to form a self-sustaining web. Some of the better known methods of bonding are overall impregnation or printing the web with intermittent or continuous straight or wavy lines for areas of binder extending generally transversely or diagonally across the web and additionally, if desired, along the web.

Where the absorbent polymer composition is used also as the binder polymer, it is applied to the fibrous starting web in an amount which is sufficient to form a self-sustaining web and provide enhanced absorbent properties upon crosslinking and suitably ranges from about 3 to about 100 wt% or more of the starting web, preferably about 10 to 50 wt% of the starting web. The impregnated web is then dried and cured. The nonwoven products are suitably dried by passing them through air ovens or the like and then through a curing oven. Typical conditions of time and temperature are well known in the art. Where a separate binder polymer is used to bond the nonwoven web, the absorbent polymer is applied to the bonded web in an amount sufficient to provide enhanced absorbent properties to the web and may range from 5 to 50 wt%, preferably 10 to 25 wt%, of the web.

The resulting web will be able to absorb many times its weight of water. The finished web could be used in sanitary products, such as diapers and feminine hygiene products, meat trays, fuel filters, seed strips, agricultural crop cover, and other applications where superabsorbents are used.

### EXAMPLE 1

This example demonstrates the preparation of a superabsorbing polymer solution.

Into a two liter jacketed glass reactor equipped with agitator and reflux condenser were charged the following ingredients:

| INITIAL CHARGE (grams) | |
|---|---|
| 1. Distilled Water | 1050 |
| 2. AIRVOL® 205 PVOH^{a} (20% soln) | 90 |
| 3. Acrylic Acid | 60 |
| 4. Vinyl Acetate | 54 |
| 5. NMA Special^{b} | 7.8 |
| 6. Dioctyl Maleate | 36 |
| 7. Potassium Persulfate | 3 |

| | |
|---|---|
| ^{a} 87-89 mole% hydrolyzed; DPn=500-600 | |
| ^{b} Aqueous N-methylolacrylamide/acrylamide mixture (26-30 wt% NMA/19-23 wt% AAm) from American Cyanamid Co. | |

| DELAY FEED (grams) | |
|---|---|
| 1. Acrylic Acid | 210 |

The 20% aqueous solution of AIRVOL 205 PVOH was added to the distilled water followed by the addition of the acrylic acid. The pH of this aqueous solution was adjusted to about 4.5 with 50% aqueous sodium hydroxide before charging it into the reactor. The vinyl acetate, NMA Special and dioctyl maleate were added under agitation of 300 rpm. The reactor was purged with nitrogen and a small stream of nitrogen was maintained during the polymerization reaction. The reaction was thermally initiated at 65°C by adding the potassium persulfate. The polymerization temperature was maintained at about 70°C.

One-half hour after the initiation, the delay feed of acrylic acid was started at a rate of 105 g/hr for 2 hours. At the middle of the acrylic acid delay, 1.5 g of potassium persulfate in 20 g water and 7.8 g NMA Special were added followed by the addition of 1.5 g potassium persulfate in 20 g of water and 30 g vinyl acetate at the end of the acrylic acid delay. The reaction was continued for an additional 3.5 hours at 70°C. A typical aqueous superabsorbent solution prepared by this procedure had the following properties:

| PROPERTIES | |
|---|---|
| 1. Total Solids (%) | 22.7 |
| 2. Brookfield Viscosity (mPa) | 2740 |
| 3. pH | 4.3 |
| 4. Maximum Absorbency Capacity | 16.0 |

The polymer composition was 64.9 wt% acrylic acid, 20.2 wt% vinyl acetate, 8.7 wt% dioctyl maleate, 1.9 wt% NMA and 4.3 wt% PVOH and had a Tg of 115°C. The maximum absorbency capacity of the polymer was determined to be 16.0g 1% aqueous saline solution/g polymer according to the following procedure:

The aqueous composition after pH adjustment to 5.5 with 10% NaOH was drizzled onto a preweighed 7 cm disc of Whatman #4 filter paper to provide about 20 wt% polymer on filter paper and dried at 300°F (149°C) for 20 minutes. The sample was placed in a sealed plastic bag in a controlled temperature and humidity room (23°C; 50% relative humidity) overnight. The equilibrated sample was sandwiched between two blank pieces of Whatman #4 filter paper and placed on a Gravimetric Absorbency Test System (GATS) apparatus (from MK Systems) with 0.07 psi weight on it to prevent the sample from floating away.

### EXAMPLE 2

This example demonstrates the preparation of a superabsorbing polymer emulsion.

Into a two liter jacketed glass reactor equipped with agitator and reflux condenser were charged the following ingredients:

| INITIAL CHARGE (grams) | |
|---|---|
| 1. Distilled Water | 1100 |
| 2. Aerosol A-102^{a} (31% active) | 20.8 |
| 3. Acrylic Acid | 50 |
| 4. Vinyl Acetate | 50 |
| 5. Ethylene Glycol Dimethacrylate | 7 |
| 6. 2-Ethylhexyl Acrylate | 25 |
| 7. Potassium Persulfate^{b} | 3 |

| | |
|---|---|
| ^{a} Disodium ethoxylated alcohol half ester of sulfosuccinic acid. | |
| ^{b} Dissolved in 30g water; added at 60°C. | |

| DELAY FEED (grams) | |
|---|---|
| 1. Acrylic Acid | 150 |
| 2. 2-Ethylhexyl Acrylate | 175 |

The 31% aqueous solution of Aerosol A-102 surfactant was added to the distilled water followed by the addition of the acrylic acid. The pH of this aqueous solution was 2.5 and was not adjusted before charging it into the reactor. The vinyl acetate, ethylene glycol dimethacrylate and 2-ethylhexyl acrylate were added under agitation of 300 rpm. The reactor was purged with nitrogen and a small stream of nitrogen was maintained during the polymerization reaction. The reaction was thermally initiated at 70°C by adding the potassium persulfate. The polymerization temperature was maintained at about 70°C.

Starting one half hour after initiation, the acrylic acid and 2-ethylhexyl acrylate delay feeds were added in two hours. At the end of the delay feeds, 2.5g potassium persulfate in 60ml water was added and the reaction was continued for 3 hours more.

| PROPERTIES | |
|---|---|
| 1. Total Solids (%) | 25.7 |
| 2. Brookfield Viscosity (mPa) | 13,150 |
| 3. pH | 2.5 |

This polymer composition was 43.8 wt% acrylic acid, 10.9 wt% vinyl acetate, 43.8 wt% 2-ethylhexyl acrylate and 1.5 wt% ethylene glycol dimethacrylate and had a Tg of 123°C. The maximum absorbency capacity of the polymer was determined to be 14.8g and 14.4g 1% aqueous saline solution/g polymer at pH 2.5 and 5, respectively, according to the procedure set forth in Example 1.

### EXAMPLE 3

This example shows the preparation of a superabsorbent polymer emulsion.

Into a two liter jacketed glass reactor equipped with agitator and reflux condenser were charged the following ingredients:

| INITIAL CHARGE (grams) | |
|---|---|
| 1. Distilled Water | 400 |
| 2. Potassium Persulfate^{a} | 5.0 |

| | |
|---|---|
| ^{a} Predissolved and added at 65°C. | |

| PREMIX DELAY FEED^{a} (grams) | |
|---|---|
| 1. Aerosol A-102 | 40 |
| 2. Distilled Water | 100 |
| 3. Acrylic Acid | 200 |
| 4. Vinyl Acetate | 50 |
| 5. 2-Ethylhexyl Acrylate | 200 |

| | |
|---|---|
| ^{a} Components 1-3 were mixed and adjusted to pH 4 with aqueous sodium hydroxide followed by the addition of components 4 and 5. | |

The polymerization temperature was maintained at 70°C and the potassium persulfate was added after commencing the delay feed which was added to the reactor at a substantially uniform rate over 3 hours. Ten minutes after the delay feed was started 9 grams NMA Special were added. One hour and twenty minutes thereafter another 9 grams NMA Special and 2 grams potassium persulfate in 20 ml water were added. At the end of the delay addition 2 g potassium persulfate in 30 grams water were added.

The resulting emulsion had the following properties:

| PROPERTIES | |
|---|---|
| 1. Total Solids (%) | 33 |
| 2. Brookfield Viscosity (mPa) | 2,840 |
| 3. pH | 3.9 |

The maximum absorbency capacity (pH=5) was 15.7 g 1% saline solution/g polymer following the procedure in Example 1.

### EXAMPLE 4

This example demonstrates the preparation of a superabsorbing polymer solution.

Into a two liter jacketed glass reactor equipped with agitator and reflux condenser were charged the following ingredients:

| INITIAL CHARGE (grams) | |
|---|---|
| 1. Distilled Water | 50 |
| 2. AIRVOL® 205 PVOH^{a} (10% soln) | 180 |
| 3. Aerosol A-102^{b} (31% active) | 40 |
| 4. SMS | 27 |
| 5. Acrylic Acid | 60 |
| 6. Vinyl Acetate | 36 |
| 7. Dioctyl Maleate | 18 |
| 8. AAEM | 6 |
| 9. Potassium Persulfate | 2.5 |

| | |
|---|---|
| ^{a} 87-89 mole% hydrolyzed; DPn = 500-600 | |
| ^{b} Disodium ethoxylated alcohol half ester of sulfosuccinic acid | |
| SMS: Sodium Methallyl Sulfonate (Hüls) AAEM: Acetoacetoxyethyl Methacrylate (Eastman) | |

| DELAY FEED (grams) | |
|---|---|
| 1. Acrylic Acid | 163 |
| 2. SMS | 20 |
| 3. NMA Special^{b} | 16 |
| 4. AAEM | 6 |
| 5. Vinyl Acetate (finishing) | 30 |
| 6. Potassium Persulfate | 3 |

| | |
|---|---|
| ^{b} Aqueous N-methylolacrylamide/acrylamide mixture (26-30 wt% NMA/19-23 wt% AAm) from American Cyanamid Co. | |

The 10% aqueous solution of AIRVOL 205 PVOH was added to the distilled water followed by the addition of the sodium methallyl sulfonate and acrylic acid. Ingredients 1-5 in the initial charge formed a solution and its pH was adjusted to about 4.5 with 50% aqueous sodium hydroxide before charging it into the reactor. The vinyl acetate, dioctyl maleate and AAEM were added under agitation of 300 rpm. The reactor was purged with nitrogen and a small stream of nitrogen was maintained during the polymerization reaction. The reaction was thermally initiated at 65°C by adding the potassium persulfate. The polymerization temperature was maintained at about 70°C.

One-half hour after the initiation, the delay feed of acrylic acid and SMS was started at a uniform rate for 2 hours. At the middle of the acrylic acid and SMS delay, 8.0 g of NMA Special, 6.0 g of AAEM and 1.5 g of potassium persulfate in water were added followed by the addition of 1.5 g potassium persulfate and 30 g vinyl acetate at the end of the acrylic acid delay. The reaction was continued for an additional 3 hours at 70° C. A typical aqueous superabsorbent solution prepared by this procedure had the following properties:

| PROPERTIES | |
|---|---|
| 1. Total Solids (%) | 45.1 |
| 2. Brookfield Viscosity (mPa) | 10,380* |
| 3. pH | 4.0 |
| 4 Vinyl Acetate (free monomer) | 0.02 |
| 5 Acrylic Acid (free monomer) | 0.00 |
| 6. Maximum Absorbency Capacity | 16.0 |
| 7. Absorbency Rate | 4.1 |

| | |
|---|---|
| * If diluted to 40% solids, the viscosity should be less than about 3000 mPa based on a graphic solid/viscosity curve correlation. | |

The polymer composition based on ingredients charged was 55.1 wt% acrylic acid, 11.6 wt% SMS, 16.3 wt% vinyl acetate, 4.5 wt% dioctyl maleate, 2.0 wt% NMA Special, 3.0 wt% AAEM and 4.5 wt% PVOH and had a Tg of 86°C. The maximum absorbency capacity of the polymer was determined to be 16.0g 1% aqueous saline solution/g polymer according to the procedure of Example 1.

### EXAMPLE 5

This example demonstrates the preparation of a superabsorbing polymer emulsion using the procedure of Example 4.

| INITIAL CHARGE (grams) | |
|---|---|
| 1. Distilled Water | 300 |
| 2. Aerosol A-102 (31% active) | 20.8 |
| 3 Airvol 523^{a} (PVOH, 10% soln) | 180 |
| 4 SMS | 45 |
| 5. Acrylic Acid | 60 |
| 6. Vinyl Acetate | 50 |
| 7. 2-Ethylhexyl Acrylate | 60 |
| 8. NMA Special | 11.8 |
| 9. Potassium Persulfate | 3 |

| | |
|---|---|
| ^{a} 87-89 mole% hydrolyzed; DPn = 1000 | |

| DELAY FEED (grams) | |
|---|---|
| 1. Acrylic Acid | 140 |
| 2. 2-Ethylhexyl Acrylate | 140 |
| 3. NMA Special | 11.8 |
| 4. Potassium Persulfate | 3 |

A typical aqueous superabsobent emulsion prepared by this method had the following properties:

| PROPERTIES | |
|---|---|
| 1. Total Solids (%) | 36.1 |
| 2. Brookfield Viscosity (mPa) | 1060* |
| 3. pH | 4.0 |
| 4 Vinyl Acetate (free monomer) | 0.05 |
| 5 Acrylic Acid (free monomer) | 0.00 |
| 6. Maximum Absorbency Capacity | 16.3 |
| 7. Absorbency Rate | 4.0 |

| | |
|---|---|
| * If concentrated to 40% solids, the viscosity should still be less than about 3000 mPa based on a graphic solid/viscosity curve correlation. | |

This polymer composition based on ingredients charged was 37.3 wt% acrylic acid, 9.2 wt% vinyl acetate, 37.3 wt% 2-ethylhexyl acrylate, 8.4 wt% SMS, 3.4 wt% PVOH, 2.2 wt% A-102 surfactant, and 2.2 wt% NMA Special and had a Tg of 125°C. The maximum absorbency capacity of the polymer was determined to be 16.3g 1% aqueous saline solution/g polymer at pH 5 according to the procedure set forth in Example 1.

### EXAMPLE 6

In this Example the effect of SMS in the copolymer is demonstrated in an aqueous polymer solution and in an aqueous polymer emulsion. The polymer solutions (Runs 1 and 2) and emulsions (Runs 3 and 4) were prepared substantially following the procedures of Examples 4 and 5 to yield polymers having the compositions set forth in Table 1.

**TABLE 1**

| **Polymer Composition** | **1 (soln)** | **2 (soln)** | **3 (emul)** | **4 (emul)** |
|---|---|---|---|---|
| Component | | | | |
| AIRVOL 205 | 4.5 | 4.5 | 3.4 | -- |
| Acrylic Acid | 67.9 | 56.9 | 37.5 | 42.6 |
| SMS | -- | 12.0 | 8.4 | -- |
| Vinyl Acetate | 21.1 | 16.8 | 9.4 | 10.6 |
| Dioctyl Maleate | 4.5 | 4.6 | -- | -- |
| 2-Ethylhexyl Acrylate | -- | -- | 37.5 | 42.6 |
| NMA (Special) | 2.0 | 2.0 | 1.7 | 1.7 |

| Composition Properties | | | | |
|---|---|---|---|---|
| Total Solids (wt%) | 22.2 | 27.8 | 39.0 | 33.0 |
| Viscosity (mPa) | 1800 | 140 | 420 | 2840 |
| pH | 5 | 4.5 | 4.2 | 3.9 |
| Tg °C | 69 | 40 | 95 | 132 |

| Absorbency Properties | | | | |
|---|---|---|---|---|
| CAP | 16.2 | 16.4 | 13.6 | 15.7 |
| Rate | 1.0 | 1.7 | 2.9 | 0.4 |

It can be seen from the data for the viscosity of the aqueous compositions that Runs 2 and 3 in which the polymer contained SMS manifested a significantly lower viscosity than the corresponding compositions Runs 1 and 4, respectively, even though they were higher in solids content.

### EXAMPLE 7

This Example demonstrates the use of acetoacetoxyethyl methacrylate as an effective latent crosslinker. The aqueous polymer compositions were prepared substantially following the procedures of Examples 4 and 5 to afford polymers having the compositions shown in Table 2.

**TABLE 2**

| **Polymer Composition** | **5 (soln)** | **6 (emul)** | **7 (soln)** | **8 (emul)** |
|---|---|---|---|---|
| Component | | | | |
| AEROSOL 102 | 3.0 | 2.2 | 3.0 | 2.3 |
| AIRVOL 205 | 4.5 | 3.4 | 4.5 | -- |
| AIRVOL 523 | -- | -- | -- | 2.8 |
| Acrylic Acid | 56.3 | 37.2 | 55.1 | 37.6 |
| SMS | 11.9 | 8.4 | 11.6 | 8.4 |
| Vinyl Acetate | 16.7 | 9.4 | 16.3 | 6.8 |
| Dioctyl Maleate | 4.6 | -- | 4.5 | -- |
| 2-Ethylhexyl Acrylate | -- | 37.2 | -- | 37.6 |
| AAEM | 3.0 | 3.2 | 3.0 | 4.5 |
| NMA (Special) | -- | -- | 2.0 | -- |

| Composition Properties | | | | |
|---|---|---|---|---|
| Total Solids (wt%) | 42.3 | 40.8 | 45.1 | 38.8 |
| Viscosity (mPa) | 2920 | 3820 | 10,380 | 1220 |
| pH | 3.9 | 4.1 | 4.0 | 4.2 |
| Tg °C | 93 | -- | 86 | -- |

| Absorbency Properties | | | | |
|---|---|---|---|---|
| CAP | 15.4 | 15.5 | 16.0 | 17.0 |
| Rate | 3.9 | 3.0 | 4.1 | 0.5 |

### STATEMENT OF INDUSTRIAL APPLICATION

The present invention provides an aqueous composition of an absorbent polymer for use in preparing superabsorbent materials such as superabsorbent fibers and nonwoven webs.

## Claims

1. An aqueous polymer composition comprising a polymer in an aqueous medium, the polymer demonstrating a maximum absorbency capacity of at least 10 g of a 1% aqueous saline solution/g polymer and consisting essentially of 25-90 wt% α,β-ethylenically unsaturated carboxylic acid monomer, 0-50 wt% allyl or methallyl sulfonic acid monomer, or both, crosslinking comonomer which is selected from the group consisting of 0.1-10 wt% latent crosslinking comonomer, 0.01-2 wt% active crosslinking comonomer, and both, and at least one softening monomer selected from the group consisting of ethylene, vinyl alkanoate, alkyl acrylate, alkyl methacrylate, monoester of a dicarboxylic acid monomer, diester of a dicarboxylic acid monomer and mixtures thereof in a sufficient amount so that the polymer has a Tg of <140°C, the softening monomer characterized as having a homopolymer that exhibits a Tg≦35°C, the aqueous composition having a viscosity less than about 3000 mPa (measured at 20% solids) and being adjusted 10 pH 4-7 with alkali metal hydroxide or alkaline earth metal hydroxide.

2. The aqueous polymer composition of Claim 1 in which the polymer contains 2-50 wt% allyl or methallyl sulfonic acid monomer, or both, and the aqueous composition has a viscosity less than about 3000 mPa (measured at 40% solids).

3. The aqueous polymer composition of Claim 1 in which the α,β-ethylenically unsaturated carboxylic acid monomer is selected from the group consisting of acrylic acid, methacrylic acid, crotonic acid, maleic acid, itaconic acid, fumaric acid and mixtures thereof.

4. The aqueous polymer composition of Claim 1 in which the viscosity is less than about 2000 mPa (measured at 20% solids).

5. The aqueous polymer composition of Claim 4 in which the softening monomers are selected from the group consisting of ethylene, C₁-C₁₂ esters of vinyl alcohol, diesters of maleic acid or fumaric acid with a C₁-C₁₆ alkanol, esters of acrylic or methacrylic acid with a C₂-C₈ alkanol and mixtures thereof.

6. The aqueous polymer composition of Claim 1 in which the polymer consists essentially of 30 to 70 wt% α,β-ethylenically unsaturated carboxylic acid monomer and has a Tg from -20° to +120°C.

7. The aqueous polymer composition of Claim 1 in which the crosslinking comonomer is a latent crosslinking comonomer.

8. The aqueous polymer composition of Claim 1 in which the crosslinking comonomer is an active crosslinking comonomer.

9. A aqueous polymer composition comprising 10 to 50 wt% of a polymer in water, the polymer demonstrating a maximum absorbency capacity of at least 10 g of a 1% aqueous saline solution/g polymer and consisting essentially of 25-90 wt% α,β-ethylenically unsaturated carboxylic acid monomer selected from the group consisting of acrylic acid, methacrylic acid, crotonic acid, maleic acid, itaconic acid and fumaric acid and mixtures thereof, 0-50 wt% sulfonic acid monomer selected from the group consisting of allylsulfonic acid, methallylsulfonic acid and both, crosslinking comonomer selected from the group consisting of 0.1-10 wt% latent crosslinking comonomer, 0.01-2 wt% active crosslinking comonomer, and both, and one or more softening monomers selected from the group consisting of ethylene, C₁-C₁₂ esters of vinyl alcohol, diesters of maleic acid or fumaric acid with a C₁-C₁₆ alkanol and esters of acrylic or methacrylic acid with a C₂-C₈ alkanol in a sufficient amount so that the polymer has a Tg <140°C, the softening monomer characterized as having a homopolymer that exhibits a Tg≦35°C, the aqueous composition having a viscosity less than about 3000 mPa (measured at 20% solids) and being adjusted to pH 4-7 with alkali metal hydroxide.

10. The aqueous polymer composition of Claim 9 which comprises 30-50 wt% of the polymer in water, the polymer consisting essentially of 30 to 70 wt% α,β-ethylenically unsaturated carboxylic acid monomer and 5 to 20 wt% sulfonic acid monomer and having a Tg from -20° to +120°C, the composition having a viscosity less than about 3000 mPa (measured at 40% solids) and being adjusted to pH 4-6 with sodium or potassium hydroxide.

11. The aqueous polymer composition of Claim 10 in which the crosslinking comonomer is a latent crosslinking comonomer selected from the group consisting of N-methylol acrylamide, acrylamidoglycolic acid, methylacrylamidoglycolate methyl ester, acrylamidobutyraldehyde dialkyl acetal, isobutoxy methyl acrylamide, chlorohydroxypropylmethacrylate, acetoacetoxyethyl (meth)acrylate and 1-(1-isocyanato-1-methylethyl)-4-(1-methyl-ethenyl) benzene

12. The aqueous polymer composition of Claim 10 in which the crosslinking comonomer is an active crosslinking comonomer selected from the group consisting of methylenebisacrylate, alkylene glycol di(meth)acrylates, 1.1.1 -trimethylolpropane dimethacrylate, divinyl benzene, vinyl (meth)acrylate, divinyl adipate, divinyl ether, triallyl (iso)cyanurate, allyl (meth)acrylate, diallyl maleate and diallyl fumarate.

13. The aqueous polymer composition of Claim 11 in which the latent crosslinking comonomer is at 1 -5 wt%.

14. The aqueous polymer composition of Claim 12 in which the active crosslinking comonomer is at 0.05-1 wt%.

15. A aqueous polymer composition comprising 10 to 50 wt% of a polymer in water, the polymer demonstrating a maximum absorbency capacity of at least 10 g of a 1% aqueous saline solution/g polymer and consisting essentially of 30 to 70 wt% acrylic acid, 0 to 20 wt% methallylsulfonic acid, crosslinking comonomer selected from the group consisting of 0.1-10 wt% N-methylol acrylamide, 0.1-10 wt% acetoacetoxyethyl(meth)acrylate, 0.01-2 wt% ethylene glycol di(meth)acrylate, 0.01-2 wt% methylenebisacrylamide, and mixtures thereof, and one or more softening monomers selected from the group consisting of ethylene, vinyl acetate, diesters of maleic acid with a C₄-C₈ alkanol and esters of acrylic or methacrylic acid with a C₂-C₈ alkanol in a sufficient amount so that the polymer has a Tg <140°C, the softening monomer characterized as having a homopolymer that exhibits a Tg≦35°C, the aqueous polymer composition having a viscosity of less than about 2000 mPa (measured at 20% solids) and being adjusted to pH 4-7 with alkali metal hydroxide.

16. The aqueous polymer composition of Claim 15 in which the polymer contains 5 to 20 wt% methallylsulfonic acid, demonstrates a maximum absorbency capacity of at least 15 g of a 1% aqueous saline solution/g polymer and has a Tg of -20° to +120°C and which composition has a viscosity of less than about 2000 mPa (measured at 40% solids) and is adjusted to pH 4-6 with sodium or potassium hydroxide.

17. The aqueous polymer composition of Claim 16 in which the softening monomers are selected from the group consisting of vinyl acetate, dioctyl maleate, 2-ethylhexyl acrylate and mixtures thereof.

18. The aqueous polymer composition of Claim 17 in which the crosslinking comonomer is N-methylolacrylamide at 1-5 wt%.

19. The aqueous polymer composition of Claim 17 in which the crosslinking comonomer is acetoacetoxyethyl(meth)acrylate at 1-5 wt%.

20. The aqueous polymer composition of Claim 17 in which the crosslinking comonomer is an ethylene glycol (meth)acrylate at 0.05-1 wt%.

21. A superabsorbent product comprising cellulosic fibers containing a sufficient amount of an absorbent polymer to provide the fibers with enhanced absorbent properties, the absorbent polymer deposited on the fibers from the aqueous polymer composition of Claim 1.

22. In a nonwoven product comprising a nonwoven web of fibers bonded together with a polymer binder, the improvement which comprises the web being bonded with a sufficient amount of a polymer to both bond the fibers and enhance the absorbent properties of the web, the polymer deposited on the fibers from the aqueous polymer composition of Claim 1.

23. In a nonwoven product comprising a nonwoven web of fibers bonded together with a polymer binder, the improvement which comprises the web also containing a sufficient amount of a polymer to enhance the absorbent properties of the web, the polymer deposited on the fibers from the aqueous polymer composition of Claim 1.
